# EUROPÄISCHE PATENTSCHRIFT

(11) **EP 1 858 478 B1**
(45) Veröffentlichungstag und Bekanntmachung des Hinweises auf die Patenterteilung: **27.02.2013**
(21) Anmeldenummer: 06723548.1
(22) Anmeldetag: 20.03.2006
(51) Int. Cl.: A61K 8/02, A61K 8/37, A61Q 15/00, A61Q 1/06, A61K 8/34

(54) **ANTITRANSPIRANT-ZUSAMMENSETZUNG AUF BASIS VON DICARBONSÄUREDIESTERN EINER C6-C18-DICARBONSÄURE MIT C12-C22- FETTALKOHOLEN**
ANTIPERSPIRANT COMPOSITION BASED ON DICARBOXYLIC ACID DIESTERS OF A C6-C18 DICARBOXYLIC ACID WITH C12-C22 FATTY ALCOHOLS
COMPOSITION ANTITRANSPIRANTE A BASE DE DIESTERS D'UN ACIDE DICARBOXYLIQUE C6-C18 ET D'ALCOOLS GRAS C12-C22

(30) Priorität: 18.03.2005 DE 102005013068
(43) Veröffentlichungstag der Anmeldung: 28.11.2007
(73) Patentinhaber: Cognis IP Management GmbH, 40589 Düsseldorf (DE)
(72) Erfinder: DIERKER, Markus, 40597 Düsseldorf (DE); WEICHOLD, Catherine, 52062 Aachen (DE); NEUSS, Michael, 50997 Köln (DE); ISSBERNER, Ulrich, 19002 Ambler (US)
(86) Internationale Anmeldenummer: PCT/EP2006/002529
(87) Internationale Veröffentlichungsnummer: WO 2006/097334

(56) Entgegenhaltungen:
- EP-A- 0 585 981
- EP-A- 0 970 998
- EP-A- 1 334 712
- FR-A- 2 857 589
- US-A1- 2002 018 760
- US-A1- 2004 223 995

## Beschreibung

### Gebiet der Erfindung

Die Erfindung betrifft wasserfreie Zusammensetzungen, die wenigstens einen Dicarbonsäurediester einer C6-C18-Dicarbonsäure mit C12-C22- Fettalkoholen enthalten und einen Schmelzpunkt von wenigstens 30°C aufweisen.

### Stand der Technik

Kosmetische Stift-Zusammensetzungen sind der Fachwelt in vielerlei Form bekannt, beispielsweise als Antitranspirantstifte, Lippenstifte oder Make-up-Stifte. Üblicherweise werden längerkettige Fettalkohole (wie z. B. Lafette® 18) oder Ricinolsäurederivate (Edenor® OSSG, Cutina® HR) als Konsistenzgeber für Stiftmassen eingesetzt. Stiftmassen auf Basis von Fettstoffen mit einem definierten Schmelzpunkt sind beispielsweise aus der EP 1 161 937 A bekannt. Die Verwendung von langkettigen, wachsartigen C16-C60-Dialkylcarbonaten in wasserfreien Antitranspirant-Zusammensetzungen ist aus der DE 101 62 049 bekannt.

Auf dem Kosmetikmarkt setzten sich in den letzten Jahren vermehrt wasserfreie Creme- und StiftPräparate durch. Im Bereich wasserfreier Antitranspirantstifte sowie sogenannten "Soft Solid"-Formulierungen finden Fettalkohole, wie beispielsweise Cetearyl-, Stearyl- und Behenylalkohol sowie Hydroxyfettsäuren, beispielsweise 12-Hydroxystearinsäure, häufigen Einsatz als sogenannte Wachsbasis. Derartige Stiftpräparate sind beispielsweise Gegenstand der US 4,822,603**,** der US 4,126,679 und der EP 117 070**.**

Wasserfreie Stiftpräparate, die flüchtige Siliconölen enthalten, haben den Nachteil, dass die dispergierten Wirkstoffe leicht zu sichtbaren Produktrückständen auf Haut und Kleidung führen. Unter Druckbelastung bei Applikation kommt es außerdem häufig zu einem Ausölen (Synärese), was die kosmetische Akzeptanz dieser Präparate beim Anwender herabsetzt. Des Weiteren hinterlässt die Fettalkohol-Basis selbst bei optimaler Auswahl der weiteren Emollients sensorisch ein unbefriedigendes Hautgefühl und führt häufig auch zu Hautreizungen.

Es bestand die Aufgabe, sensorisch bessere Konsistenzgeber für wasserfreie Stiftzusammensetzungen zur Verfügung zu stellen, die den Stiftmassen die nötige Härte verleihen und glatte Stiftoberflächen mit schönem Glanz liefern, die weniger zum "Bröckeln" neigen als Produkte des Standes der Technik.

Überraschenderweise wurde gefunden, dass dies gezielt durch bestimmte Dicarbonsäurediester erreicht werden kann.

### Beschreibung der Erfindung

Gegenstand der vorliegenden Anmeldung sind daher wasserfreie kosmetische Zusammensetzungen mit einem Schmelzpunkt von wenigstens 30°C enthaltend
(a) wenigstens einen Dicarbonsäurediester einer C6-C18-Dicarbonsäure mit C12-C22-Fettalkoholen
(b) wenigstens eine Ölkomponente
(c) wenigstens ein anorganisches adstringierendes Salz, einen Deodorantwirkstoff oder ein Pigment.

Als wasserfrei werden im Sinne der Erfindung Zusammensetzungen bezeichnet, die weniger als 5 Gew.-% Wasser (Kristallwasser nicht mit einbezogen), vorzugsweise weniger als 2 Gew.-% Wasser und insbesondere weniger als 1 Gew.-% Wasser enthalten. Ein Restanteil an Wasser kann rohstoffbedingt und daher unvermeidbar sein.

Die spezifischen Disäurediester sind sehr gute Konsistenzgeber in wasserfreien kosmetischen Formulierungen. Erfindungsgemäß kann ein einziger Dicarbonsäurediester eingesetzt werden oder ein Gemisch aus Dicarbonsäurediestem oder ein Gemisch der Dicarbonsäurediester mit den marktüblichen Konsistenzgebem wie Fettalkohole, etc. Besonders geeignet sind diese Ester für den Applikationsbereich der AP/Deo-Stifte. Sie verleihen den Stiftmassen die nötige Härte und eine glatte Stiftoberfläche mit einen schönen Glanz, die weniger zum "Bröckeln" neigt. In einer besonders bevorzugten Ausführungsform sind die Stiftmassen frei von Silikonölen, insbesondere leichtflüchtigen Silikonölen wie Cyclomethicone.

Für die erforderliche Härte ist im Vergleich zu Fettalkoholen, wie Lanette® 18, ein geringerer Anteil der spezifischen Dicarbonsäurediester in der Stiftformulierung nötig. Die spezifischen Dicarbonsäurediester sind mit zahlreichen Emollients kompatibel, wie beispielsweise Cetiol® OE, Cetiol® CC als auch mit Cyclomethicon oder Kohlenwasserstoffen wie Arlamol® HD, Synfluid® PAO. Es lassen sich auch silikonfreie Stiftmassen und Deostofte herstellen. Die spezifischen Dicarbonsäurediester werden nicht durch starke Lewis Säuren hydrolysiert, wie sie in der Regel in Antitranspirantformulierungen eingesetzt werden (z.B. Aluminiumchlorid, Aluminiumchlorohydrat, Zirkonium-Salze und Mischungen davon). Dies trifft auch auf den Einsatz in wasserhaltigen Antitranspirant-Formulierungen zu. Im Vergleich zu den üblichen Verdickem, wie Lanette® 18 oder Cutina® HR (gehärtetes Ricinusöl), verfügen die Disäurediester über keine freien OH-Gruppen, die zu Reaktionen mit den Lewis-Säuren führen könnten.

Die Dicarbonsäure-Komponente der Dicarbonsäurediester ist ausgewählt aus den C6-C18-Dicarbonsäuren. Hierzu zählen als wichtigste Vertreter Adipinsäure, Azealainsäure, Sebacinsäure, Dodecandisäure und Octadecandisäure. In einer bevorzugten Ausführungsform der Erfindung ist die Dicarbonsäure-Komponente ausgewählt ist aus den unverzweigten, gesättigten C6-C18-Dicarbonsäuren, vorzugsweise aus den unverzweigten, gesättigten C9-C18-Dicarbonsäuren und insbesondere den unverzweigten, gesättigten C12-C18-Dicarbonsäuren.

Als Alkohol-Komponente der Ester eignen sich die üblichen C12-C22-Fettalkohole, beispielsweise Dodecanol, Tridecanol, Tetradecanol, Pentadecanol, Hexadecanol, Heptadecanol, Octadecanol, Octadecenol, Nonadecanol, Eicosanol, Eicosenol, Heneicosanol, Docosanol und Docosenol. Auch technische Gemische von primären Alkoholen, wie sie mittels üblicher fettchemischer Methoden aus natürlichen Fetten und Ölen erhältlich sind, lassen sich erfindungsgemäß einsetzen. Erfindungsgemäß bevorzugt ist der Einsatz von unverzweigten, gesättigten C16-C22-Fettalkoholen, insbesondere C16-C18- Fettalkoholen.

In einer bevorzugten Ausführungsform der Zusammensetzung ist ein Dicarbonsäurediester mit einem Schmelzpunkt von wenigstens 50 °C enthalten, vorzugsweise von 50 - 55 °C.

Als Komponente (c) ist wenigstens anorganisches adstringierendes Salz, einen Deodorantwirkstoff oder ein Pigment enthalten, je nachdem ob es sich um eine Stiftmasse im AP/Deo-Bereich oder der dekorativen Kosmetik handelt.

### Pigmente

Geeignet sind alle üblicherweise in Lippenstiften, Lidschattenstiften und Make-up-Stiften eingesetzten Oxide und Pigmente, wie z.B. Titandixoid, Eisenoxide, Zinkoxid, Glimmerpigmente, Mica-Pigmente oder Perlglanzpigmente, deren optischer Effekt auf der Kombination aus Transparenz und Interferenz beruht. Letztere sind nach dem Schicht-Substrat-Prinzip aus der optisch neutralen Trägersubstanz Glimmer und einer optisch aktiven Metalloxid-Schicht aufgebaut (Schicht-Substrat-Pigmente). Entscheidend für die Ausbildung des Perlglanzeffektes oder Interferenzeffektes ist eine genügend große Differenz zwischen der Brechzahl des Glimmers (n =1,5) und der des Metalloxids (n = 2,5-2,7). Man unterscheidet zwischen Titandioxid-Glimmer-Pigmenten, Titandioxid-Eisenoxid-Glimmer-Pigmenten, Eisenoxid-Glimmer-Pigmenten.

Metalloxid-Glimmer-Pigmente sind trockene Pulver mit einer Dichte von ca. 3 g/m3. Sie sind im allgemeinen bis 800°C temperaturbeständig. Der Perlglanzeffekt wird bestimmt durch die Art des Metalloxids und dessen Schichtdicke auf dem Glimmer. Die Pigmente sind mehr oder weniger transparent. Ihr Glanz und Deckvermögen sind abhängig von der Teilchengrößenverteilung, z.B. <15 µm, 5-25 µm, 10-60 µm oder 20-100 µm.

Titandioxid-Glimmer-Pigmente bilden die wichtigste Gruppe unter den Perlglanzpigmenten. Auf dem Glimmer-Plättchen befindet sich eine 40-60 nm dicke Titandioxid-Schicht der Anatas-Modifikation oder Rutil-Modifikation. Die Pigmente sind weiß und führen zu feinen, seidigen Effekten (feine Teilchen) oder groben Glitzereffekten (grobe Teilchen). Pigmente mit höherer Schichtdicke des Titandioxids (60-160 nm) wirken als Interferenzpigmente.

Bei den Titandioxid-Eisenoxid-Glimmer-Pigmenten befindet sich auf der Titandioxid-Schicht noch eine Schicht Eisenoxid. Es resultieren - in Abhängigkeit von der Schichtdicke der Metalloxide - hellgoldene, rötlich-goldene oder grünlich-goldene Farbglanzpigmente, deren Deckvermögen durch das Eisenoxid deutlich erhöht ist.

Wird der Glimmer direkt mit Eisenoxid beschichtet, erhält man braune bis rotbraune Eisenoxid-Glimmer-Pigmente mit nochmals verbessertem Deckvermögen. Je nach Schichtdicke des Eisenoxids variieren die Farbtöne zwischen bronze, kupfer, rot bis rotgrün.

Titandioxid-Eisentitanat-Glimmer-Pigmente sind silbergrau, blaugrau und anthrazitfarben. Auch Rußeinschlüsse in die Titandioxid-Schicht führen zu grauen Pigmenten. Weitere Farbtöne lassen sich durch Belegung der Titandioxid-Schicht mit Eisenblau oder organischen Substanzen wie Karminrot erzielen.

Ein weiterer Gegenstand der Anmeldung ist die Verwendung von Dicarbonsäurediester einer C6-C18-Dicarbonsäure mit C12-C22- Fettalkoholen in Stiftmassen für die dekorative Kosmetik. Hierzu zählen Make-up-Stifte, Lidschattenstifte und insbesondere Lippenstifte.

### Anorganisches adstringierendes Salz: Antitranspirant-Wirkstoff

Als Antitranspirant-Wirkstoff geeignet sind prinzipiell alle anorganischen adstringierend wirkenden Salze, die eine Schweißreduktion bewirken. Erfindungsgemäß handelt es sich vorzugsweise um adstringierende Aluminium-Verbindungen, Aluminium-Zirkonium-Verbindungen oder Zinksalze. Hierzu zählen beispielsweise Aluminiumchlorid, Aluminiumchlorhydrat, Aluminiumdichlorhydrat, Aluminiumsesquichlorhydrat und deren Komplexverbindungen, z.B. mit 1,2-Propylenglycol, Aluminiumhydroxyallantoinat, Aluminium-chloridtartrat, Aluminium-Zirkonium-Trichlorohydrat, Aluminium-Zirkonium-Tetrachlorohydrat, Alumini-um-Zirkonium-Pentachlorohydrat und deren Komplexverbindungen z.B. mit Aminosäuren wie Glycin. Vorzugsweise werden Aluminiumchlorhydrat, Aluminium-Zirkonium-Tetrachlorohydrat, Aluminium-Zirkonium-Pentachlorohydrat und deren Komplexverbindungen eingesetzt. Besonders bevorzugt ist der Einsatz von Verbindungen, die unter der Bezeichnung Locron^{®} P und Rezal^{®} 36 GP im Handel sind.

Eine bevorzugte Ausführungsform der Erfindung ist eine Zusammensetzung enthaltend (a) 5 - 40 Gew.% wenigstens eines Diesters einer C6-C18-Dicarbonsäure mit C12-C22- Fettalkoholen, (b) 30 - 80 Gew.% wenigstens einer Ölkomponente und (c) 10-40 Gew.-% wenigstens eines Antitranspirantwirkstoffs. Besonders bevorzugt ist eine Zusammensetzung enthaltend (a) 8 - 25 Gew.-% wenigstens eines Diesters einer C6-C18-Dicarbonsäure mit C12-C22- Fettalkoholen, (b) 40 - 70 Gew.-% wenigstens einer Ölkomponente und (c) 15 - 30 Gew.-% wenigstens eines Antitranspirantwirkstoffs und insbesondere (a) 12 - 20 Gew.% wenigstens eines Diesters einer C6-C18-Dicarbonsäure mit C12-C22- Fettalkoholen, (b) 50 - 60 Gew.-% wenigstens einer Ölkomponente und (c) 15 - 25 Gew.-% wenigstens eines Antitranspirantwirkstoffs.

Ein weiterer Gegenstand der Anmeldung ist die Verwendung von Dicarbonsäurediester einer C6-C18-Dicarbonsäure mit C12-C22- Fettalkoholen in Antitranspirant-Zusammensetzungen. Diese können sowohl wasserhaltig als auch "wasserfrei" im Sinne der Erfindung sein. Gegenstand der Anmelödung ist insbesondere die Verwendung von Dicarbonsäurediester einer C6-C18-Dicarbonsäure mit C12-C22-Fettalkoholen zur Herstellung wasserfreier Antitranspirant-Zusammensetzungen. Ein weiterer Gegenstand der Anmeldung ist die Verwendung von Dicarbonsäurediester einer C6-C18-Dicarbonsäure mit C12-C22- Fettalkoholen zur Verbesserung der Konsistenz von Antitranspirant- und Deostiften.

Vorzugsweise weisen die erfindungsgemäßen Zubereitungen bei 23° C und einer Eindringzeit von 5 Sekunden eine Eindringtiefe von weniger als 5mm, vorzugsweise 2,5 - 4mm auf (Penetrometer PNR 10 Petrotest; Petrotest Instruments GmbH & Co KG; Mikrokonus: 5.0 g; Fallstab: 47.5 g; Messtemperatur: 23° C; Meßdauer 5 Sekunden). Die Eindringtiefe ist somit ein Maß für die "Härte" oder Konsistenz der Masse. Je kleiner der Wert für die Eindringtiefe, desto "härter" ist die Masse.

### Deodorantwirkstoffe

Als Deodorant-Wirkstoffe ist eine Reihe sehr verschiedener Verbindungsklassen geeignet. Vorzugsweise werden diese in Kombination mit Antitranspirant-Wirkstoffen eingesetzt, um eine optimale Gesamtleistung, also eine Kaskaden-Effekt mit langanhaltender Wirkung, zu erreichen.

### Esteraseinhibitoren

Beim Vorhandensein von Schweiß im Achselbereich werden durch Bakterien extrazelluläre Enzyme - Esterasen, vorzugsweise Proteasen und/oder Lipasen - gebildet, die im Schweiß enthaltene Ester spalten und dadurch Geruchsstoffe freisetzen. Zu den Stoffen, die die Enzymaktivität inhibieren und somit die Geruchsbildung reduzieren, zählen Trialkylcitrate, wie Trimethylcitrat, Tripropylcitrat, Triisopropylcitrat, Tributylcitrat und insbesondere Triethylcitrat (Hydagen® CAT, Cognis GmbH, Düsseldorf/FRG). Wahrscheinlich wird dabei durch Spaltung des Citronensäureesters die freie Säure freigesetzt, die den pH-Wert auf der Haut soweit absenkt, dass die Enzyme durch Acylierung inaktiviert werden. Weitere Stoffe, die als Esteraseinhibitoren in Betracht kommen, sind Sterolsulfate oder - phosphate, wie beispielsweise Lanosterin-, Cholesterin-, Campesterin-, Stigmasterin- und Sitosterinsulfat bzw. -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester sowie Zinkglycinat.

Die erfindungsgemäßen Mittel können die Esteraseinhibitoren in Mengen von 0,01 bis 20, vorzugsweise 0,1 bis 10 Gew.% und insbesondere 0,5 bis 5 Gew.% bezogen auf die Gesamtzusammensetzung enthalten.

### Bakterizide bzw. bakteriostatische Wirkstoffe

Typische Beispiele für geeignete bakterizide bzw. bakteriostatische Wirkstoffe sind Chitosan und Phenoxyethanol. Besonders wirkungsvoll hat sich auch 5-Chlor-2-(2,4-dichlorphenoxy)-phenol erwiesen, das unter der Marke Irgasan® von der Ciba-Geigy, Basel/CH vertrieben wird.

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren. Als keimhemmende Mittel sind grundsätzlich alle gegen grampositive Bakterien wirksamen Stoffe geeignet, wie z.B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phe-nol, 2-Benzyl-4-chlorphenol, 3-(4-Chlorphenoxy)-1,2-propandiol, 3-lod-2-propinylbutylcarbamat, Chlor-hexidin, 3,4,4'-Trichlorcarbanilid (TTC), antibakterielle Riechstoffe, Thymol, Thymianöl, Eugenol, Nel-kenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z.B. Salicyl-säure-n-octylamid oder Salicylsäure-n-decylamid.

Die erfindungsgemäßen Mittel können die bakterizide/bakteriostatische bzw. keimhemmenden Wirkstoffe in Mengen von 0,01 bis 10, vorzugsweise 0,1 bis 5 Gew.-% und insbesondere 0,5 - 2 Gew.-% enthalten.

Neben diesen Deodorant-Wirkstoffen werden häufig zusätzlich Geruchsabsorber eingesetzt, die geruchsbildende Verbindungen aufnehmen und weitgehend "festhalten" können, aber keine Wirksamkeit gegen Bakterien haben. Sie senken den Partialdruck der einzelnen Komponenten und verringern so auch ihre Ausbreitungsgeschwindigkeit. Geruchsabsorber sollten die Duftnote eines Parfums nicht verändern. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z.B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

Neben den Deodorant-Wirkstoffen werden häufig zusätzlich auch schweißabsobierende Substanzen eingesetzt, wie modifizierte Stärke, z.B. Dry Flo^{®} Plus (National Starch), Silikate, Talkum, Bentonit, Monmorillonit, Hectorit und andere Substanzen ähnlicher Modifikation, die zur Schweißabsorption geeignet erscheinen.

### Ölkörper

Die erfindungsgemäße Zusammensetzung enthält wenigsten eine Ölkomponente. Unter Ölkomponenten sind erfindungsgemäß bei 20 °C flüssige, mit Wasser bei 25 °C nicht mischbare Stoffe oder Gemische von Stoffen zu verstehen. Hierzu gehören z.B. bei 20°C flüssige Glyceride, Kohlenwasserstoffe, Siliconöle, Esteröle, Dialkyl(en)ether, Dialkyl(en)carbonate oder beliebige Gemische davon. Die Ölkörper sind in den erfindungsgemäßen Zusammensetzungen üblicherweise in einer Gesamtmenge von 30-80 Gew.-%, bevorzugt in einer Menge von 40 - 70 und insbesondere in Mengen von 50 - 60 Gew.-% bezogen auf die Gesamtzusammensetzung enthalten.

Zu den als Ölkörper erfindungsgemäß einsetzbaren Glyceriden zählen bei 20 °C flüssige Fettsäureester des Glycerins, die natürlicher (tierischer und pflanzlicher) oder synthetischer Herkunft sein können. Man unterscheidet zwischen Mono-, Di- und Triglyceriden. Es handelt sich um bekannte Stoffe, die nach einschlägigen Verfahren der präparativen organischen Chemie hergestellt werden können. Synthetisch hergestellte Glyceride sind üblicherweise Mischungen von Mono-, Di- und Triglyceriden, die durch Umesterung der entsprechenden Triglyceride mit Glycerin oder durch gezielte Veresterung von Fettsäuren erhalten werden. Als Fettsäure sind erfindungsgemäß C₆-C₂₄-Fettsäuren, und unter diesen C₆-C₁₈-Fettsäuren, und insbesondere C₈-C₁₈-Fettsäuren bevorzugt geeignet. Die Fettsäuren können verzweigt oder unverzweigt, gesättigt oder ungesättigt sein. Erfindungsgemäß geeignet ist beispielsweise die Verwendung bei 20 °C flüssiger Glyceride pflanzlicher Herkunft, insbesondere von Cocoglyceriden, einer Mischung aus vorwiegend Di- und Triglyceriden mit C₈-C₁₈-Fettsäuren, die beispielsweise unter der Bezeichnung Myritol^{®} 331 von der Cognis Deutschland GmbH & Co KG vertrieben werden. Ebenso geeignet ist die Verwendung von Myritol^{®} 312 (C₈/C₁₀-Triglyceride), Cegesoft^{®} PS 17, Cegesoft^{®} GPO; Cegesoft^{®} PFO und Cegesoft^{®} PS 6.

Eine erfindungsgemäß bevorzugte Ausführungsform der Erfindung enthält wenigstens einen Ölkörper ausgewählt aus der Gruppe der bei Dialkylether, der Dialkylcarbonate, Kohlenwasserstoffe oder einem beliebigen Gemisch dieser Substanzen. Diese verleihen den Zusammensetzungen bei Applikation besonders gute pflegende und sensorische Eigenschaften und ein angenehm trockenes Hautgefühl. Ebenso bevorzugt ist die Kombination eines Dialkylethers mit Cyclohexanderivaten. Eine besonders bevorzugte Ölkörper-Kombination für die erfindungsgemäßen Zusammensetzungen enthält Cetiol^{®} OE und Cetiol^{®} S.

Als Ölkörper kommen auch bei 20° C flüssige Guerbetalkohole auf Basis von Fettalkoholen mit 6 bis 18, vorzugsweise 8 bis 10 Kohlenstoffatomen, wie beispielsweise Eutanol^{®} G, in Frage. Auch flüssige Ester von linearen, gesättigten oder ungesättigten C₆-C₂₂-Fettsäuren mit linearen oder verzweigten, gesättigten oder ungesättigten C₆-C₂₂-Fettalkoholen bzw. Ester von verzweigten C₆-C₁₃-Carbonsäuren mit linearen oder verzweigten, gesättigten oder ungesättigten C₆-C₂₂-Fettalkoholen sind erfindungsgemäß als Ölkörper einsetzbar.

Unter den bei 20°C flüssigen Wachsestem seien expemplarisch folgende typische Vertreter genannt: Decyloleat (Cetiol^{®} V), Cococaprylate/-caprat (Cetiol^{®} SN), Hexyllaurat (Cetiol^{®} A), Myristylisostearat, Myristyloleat, Cetylisostearat, Cetyloleat, Stearylisostearat, Isostearylmyristat, Iso-stearylpalmitat, Isostearylstearat, Isostearylisostearat, Isostearyloleat, Isostearylbehenat, Iso-stearyloleat, Oleylmyristat, Oleylpalmitat, Oleylstearat, Oleylisostearat, Oleyloleat (Cetiol^{®}RDAB), Oleylbehenat, Oleylerucat (Cetiol^{®} J 600), Behenylisostearat, Erucylisostearat, Erucyloleat. Geeignet sind auch Ester von linearen C₆-C₂₂-Fettsäuren mit 2-Ethylhexanol (Cetiol^{®} 868), Ester von C₁₈-C₃₈-Alkylhydroxycarbonsäuren mit linearen oder verzweigten C₆-C₂₂-Fettalkoholen, Ester von linearen und/oder verzweigten Fettsäuren mit mehrwertigen Alkoholen (wie z.B. Propylenglycol, Dimerdiol oder Trimertriol) sowie Ester von C₆-C₂₂-Fettalkoholen und/oder Guerbetalkoholen mit aromatischen Carbonsäuren, insbesondere Benzoesäure.

Zu den erfindungsgemäß einsetzbaren Ölkörpem zählen auch bei 20 °C flüssige natürliche und synthetische, aliphatische und/oder naphthenische Kohlenwasserstoffe, wie z.B. Squalan, Squalen, Paraffinöle, Isohexadecan, Isoeicosan oder Polydecene sowie Dialkylcyclohexane (Cetiol®S).

### Wachse

In einer weiteren bevorzugten Ausführungsform enthalten die erfindungsgemäßen Zusammensetzungen wenigstens ein weiteres Wachs. Dieses trägt zur Optimierung der sensorischen Eigenschaften, der Konsistenz und der Stabilität der Stifte bei. Als weitere Wachse (Definition vgl.: Römpp Chemie Lexikon) können erfindungsgemäß alle natürlichen und künstlich gewonnen Substanzen mit wachsartiger Konsistenz eingesetzt werden. Hierzu gehören u.a. Fette (Triglyceride), Mono- und Diglyceride, Wachse, Fett- und Wachsalkohole, Fettsäuren, Ester von Fettalkoholen sowie Fettsäureamide oder beliebige Gemische dieser Substanzen. Sie können in den erfindungsgemäßen Zusammensetzungen in einer Gesamtmenge von 0,1 - 40 Gew.-% enthalten sein. Bevorzugt sind Mengen von 1 - 30 Gew.% und insbesondere 5 - 20 Gew.-% bezogen auf die Gesamtzusammensetzung. Vorzugsweise werden längerkettige Fettalkohole (wie z. B. Lanette® 18 und Lanette® 22) oder Ricinolsäurederivate (Edenor® OSSG, Cutina® HR) als zusätzliche Wachse für die erfindungsgemäßen Stiftmassen eingesetzt.

### Beispiele

**Tabelle 1: Schmelzpunkte der Dicarbonsäurediester**

| | **Verwendete Alkoholkomponente** | | | |
|---|---|---|---|---|
| **Dicarbonsäure** | | **Lorol Spezial** | **Stenol 16-18** | **Stenol C1822AT** |
| Adipinsäure | C 6 | 33°C | 51-55°C | 53-30°C |
| Azelainsäure | C 9 | 35-36,5°C | 54-55°C | 57-66°C |
| Sebacinsäure | C 10 | 37-39°C | 53-58°C | 65-68°C |
| Dodecandisäure | C 12 | 42-44°C | 59-61°C | 64-68°C |
| Octadecansäure | C 18 | - | 66-69°C | 73-76°C |

**Tabelle 2: Säurezahlen der Dicarbonsäurediester**

| | **Verwendete Alkoholkomponente** | | | |
|---|---|---|---|---|
| **Dicarbonsäure** | | **Lorol Spezial** | **Stenol 16-18** | **Stenol C1822AT** |
| Adipinsäure | C 6 | 0,8 | 1,0 | 0,8 |
| Azelainsäure | C 9 | 0,8 | 0,8 | 1,2 |
| Sebacinsäure | C 10 | 1,0 | 0,5 | 0,8 |
| Dodecandisäure | C 12 | 0,9 | 1,0 | 0,3 |
| Octadecansäure | C 18 | - | 0,5 | 0,4 |

**Tabelle 3: OH-Zahlen der Dicarbonsäurediester**

| | **Verwendete Alkoholkomponente** | | | |
|---|---|---|---|---|
| **Dicarbonsäure** | | **Lorol Spezial** | **Stenol 16-18** | **Stenol C1822AT** |
| Adipinsäure | C 6 | 3,2 | 5,0 | 2,0 |
| Azelainsäure | C 9 | 1,4 | 1,1 | 0,6 |
| Sebacinsäure | C 10 | 1,2 | 3,6 | 2,1 |
| Dodecandisäure | C 12 | 1,2 | 0,3 | 0,9 |
| Octadecansäure | C 18 | - | 5,0 | 2,0 |

**Tabelle 4: Allgemeine Zusammensetzung eines Antitranspirantstiftes**

| | **1** | **2** |
|---|---|---|
| Dicarbonsäurediester | 12,00 | 20,00 |
| DC® 245 | 37,00 | 33,00 |
| Cetiol® OE | 10,00 | 9,00 |
| Cetiol® S | 18,00 | 15,00 |
| Rezal® 36GP | 23,00 | 23,00 |

**Tabelle 5: Stifthärten, vgl. Zusammensetzung 1 in Tabelle 4**

| | | **Verwendente Alkoholkomponente** | | |
|---|---|---|---|---|
| | | Lorol Spezial | Stenol 16-18 | Stenol C1822 AT |
| | | C₁₂₋₁₄ | C₁₆₋₁₈ | C₁₈₋₂₂ |
| Adipinsäure | C₆ | - | 4,70 | 4,47 |
| Azelainsäure | C₉ | - | - | 4,50 |
| Sebacinsäure | C₁₀ | - | 5,51 | 4,10 |
| Dodecandisäure | C₁₂ | 7,45 | 4,87 | 3,38 |
| Octadecandisäure | C₁₈ | - | - | 3,69 |

**Tabelle 6: Stifthärten, vgl. Zusammensetzung 2 in Tabelle 4**

| | | **Verwendete Alkoholkomponente** | | |
|---|---|---|---|---|
| | | Lorol Spezial | Stenol 16-18 | Stenol C1822 AT |
| | | C12-14 | C₁₆₋₁₈ | C₁₈₋₂₂ |
| Adipinsäure | C₆ | - | 2,60 | 2,36 |
| Azelainsäure | c₉ | - | - | 2,38 |
| Sebacinsäure | C₁₀ | 8,86 | 4,70 | 1,99 |
| Dodecandisäure | C₁₂ | 2,80 | 2,71 | 2,63 |
| Octadecandisäure | C₁₈ | - | - | 1,80 |

**Tabelle 7: Antitranspirant/Deostifte**

| | **3** | **4** | **5** |
|---|---|---|---|
| Adipinsäure-di-12/14-ester | 4,00 | 4,00 | |
| Adipinsäure-di-Lanette O | 16,00 | | |
| Adipinsäure-di-18/22-ester | | 16,00 | |
| Azelainsäure-di-12/14-ester | | | 4,00 |
| Sebazinsäure-18/22-ester | | | 16,00 |
| Dow Coming® 245 | 33,00 | 33,00 | 33,00 |
| Cetiol® OE | 9,00 | 9,00 | 9,00 |
| Cetiol® S | 15,00 | 15,00 | 15,00 |
| Rezal® 36GP | 23,00 | 23,00 | 23,00 |

| **Härte** | | | |
|---|---|---|---|
| 1. Tag | 4,55 | 3,59 | 3,77 |

**Tabelle 8:**

| **Inhaltsstoffe** | **1** | **2** | **3** |
|---|---|---|---|
| Dodecandisäure-16/18-diester | 8,00 | 8,00 | 16,00 |
| Cutina® HR | 4,00 | | |
| Edenor® OSSG | | 4,00 | |
| Lanette® 18 | | | 4,00 |
| DC® 245 | 37,00 | 37,00 | 33,00 |
| Cetiol® OE | 10,00 | 10,00 | 9,00 |
| Cetiol® S | 18,00 | 18,00 | 15,00 |
| Rezal® 36GP | 23,00 | 23,00 | 23,00 |
| Härte | | | |
| Anfang | 8,38 | 4,43 | 3,07 |
| Struktur Anfang | | | |
| Oberfläche | matt.matschig | glänzend,weiß | glänzend, |
| Abrieb | bröckelig sehr weiß, | leicht bröckelig, | leicht weiß, |
| geschnittene Fläche | matt grob | fein weiß | fein matt |

**Tabelle 9:**

| **Inhaltsstoffe** | **4** | **5** | **6** |
|---|---|---|---|
| Adipinsäure-di-Lanette O 16/18 | 8,00 | 8,00 | 16,00 |
| Cutina® HR | 4,00 | | |
| Edenor® OSSG | | 4,00 | |
| Lanette® 18 | | | 4,00 |
| DC® 245 | 37,00 | 37,00 | 33,00 |
| Cetiol® OE | 10,00 | 10,00 | 9,00 |
| Cetiol® S | 18,00 | 18,00 | 15,00 |
| Rezal® 36GP | 23,00 | 23,00 | 23,00 |
| Härte | | | |
| Anfang | 5,34 | 5,86 | 5,6 |
| Struktur Anfang | | | |
| Oberfläche | matt, sehr weiß viel | glänzend, | hängt am Deckel |
| Abrieb | sehr weiß, viel Abrieb, bröckelig | leicht weiß | leicht weiß |
| geschnittene Fläche | bröckelig grob | bröckelig fein | matt fein |

**Tabelle 10:**

| **Inhaltsstoffe** | **7** | **8** | **9** |
|---|---|---|---|
| Azelainsäure-di-16/18-ester | 8,00 | 8,00 | 16,00 |
| Cutina® HR | 4,00 | | |
| Edenor® OSSG | | 4,00 | |
| Lanette® 18 | | | 4,00 |
| DC® 245 | 37,00 | 37,00 | 33,00 |
| Cetiol® OE | 10,00 | 10,00 | 9,00 |
| Cetiol® S | 18,00 | 18,00 | 15,00 |
| Rezal® 36GP | 23,00 | 23,00 | 23,00 |
| Härte | | | |
| Anfang | 4,19 | 4,56 | 2,91 |
| Struktur Anfang | | | |
| Oberfläche | matt, | matt, | leicht glänzend |
| Abrieb | weiß bröckelig | weiß bröckelig | weiß leicht bröckelig |
| geschnittene Fläche | fein bröckelig | fein matt | fein matt |

**Tabelle 11:**

| **Inhaltsstoffe** | **10** | **11** | **12** |
|---|---|---|---|
| Adipinsäure-di-18/22-ester | 8,00 | 6,00 | 16,00 |
| Cutina® HR | 4,00 | | |
| Edenor® OSSG | | 4,00 | |
| Lanette® 18 | | | 4,00 |
| DC® 245 | 37,00 | 37,00 | 33,00 |
| Cetiol® OE | 10,00 | 10,00 | 9,00 |
| Cetiol® S | 18,00 | 18,00 | 15,00 |
| Rezal® 36GP | 23,00 | 23,00 | 23,00 |
| Härte | | | |
| Anfang | 38,01 | 4,38 | 2,87 |
| Struktur Anfang | | | |
| Oberfläche | matschig | glänzend | glänzend |
| Abrieb | sehr weiß | leicht weiß creamig | weiß creamig |
| geschnittene Fläche | bröckelig grob matschig | fein matt | fein matt |

**Tabelle 12:**

| **Inhaltsstoffe** | **13** | **14** | **15** |
|---|---|---|---|
| Azelainsäure-di-18/22-ester | 8,00 | 8,00 | 16,00 |
| Cutina® HR | 4,00 | | |
| Edenor® OSSG | | 4,00 | |
| Lanette® 18 | | | 4,00 |
| DC® 245 | 37,00 | 37,00 | 33,00 |
| Cetiol® OE | 10,00 | 10,00 | 9,00 |
| Cetiol® S | 18,00 | 18,00 | 15,00 |
| Rezal® 36GP | 23,00 | 23,00 | 23,00 |
| Härte | | | |
| Anfang | 6,38 | 4,33 | 4,14 |
| Struktur Anfang | | | |
| Oberfläche | leicht bröckelig | teil glänzend | glänzend |
| Abrieb | sehr weiß matschig | leicht weiß | weiß leicht bröckelig |
| beschnittene Fläche | fein bröckelig | fein matt | fein matt |

**Tabelle 13:**

| **Inhaltsstoffe** | **16** | **17** | **18** |
|---|---|---|---|
| Sebacinsäure-di18/22-ester | 8,00 | 8,00 | 16,00 |
| Cutina® HR | 4,00 | | |
| Edenor® OSSG | | 4,00 | |
| Lanette® 18 | | | 4,00 |
| DC® 245 | 37,00 | 37,00 | 33,00 |
| Cetiol® OE | 10,00 | 10,00 | 9,00 |
| Cetiol® S | 18,00 | 18,00 | 15,00 |
| Rezal® 36GP | 23,00 | 23,00 | 23,00 |
| Härte | | | |
| Anfang | 3,99 | 4,27 | 3,54 |
| Struktur Anfang | | | |
| Oberfläche | matt | teil glänzend | teil glänzend |
| Abrieb | sehr weiß matschig | weiß leicht bröckelig | weiß leicht bröckelig |
| geschnittene Fläche | bröckelig grob | fein matt | fein matt |

**Tabelle 14:**

| **Inhaltsstoffe** | **19** | **20** | **21** |
|---|---|---|---|
| C12-Disäure-di-18/22-ester | 8,00 | 8,00 | 16,00 |
| Cutina® HR | 4,00 | | |
| Edenor® OSSG | | 4,00 | |
| Lanette® 18 | | | 4,00 |
| DC® 245 | 37,00 | 37,00 | 33,00 |
| Cetiol® OE | 10,00 | 10,00 | 9,00 |
| Cetiol® S | 18,00 | 18,00 | 15,00 |
| Rezal® 36GP | 23,00 | 23,00 | 23,00 |
| Härte | | | |
| Anfang | 4,51 | 4,61 | 3,34 |
| Struktur Anfang | | | |
| Oberfläche | matt | matt teil glänzend | glänzend |
| Abrieb | weiß leicht bröckelig | weiß leicht bröckelig | weiß leicht bröckelig |
| geschnittene Fläche | fein matt | fein matt | fein matt |

**Tabelle 15:**

| **Inhaltsstoffe** | **22** | **23** | **24** |
|---|---|---|---|
| DCA:O-di-Stenol18/22-ester | 8,00 | 8,00 | 16,00 |
| Cutina® HR | 4,00 | | |
| Edenor® OSSG | | 4,00 | |
| Lanette® 18 | | | 4,00 |
| DC® 245 | 37,00 | 37,00 | 33,00 |
| Cetiol® OE | 10,00 | 10,00 | 9,00 |
| Cetiol® S | 18,00 | 18,00 | 15,00 |
| Rezal® 36GP | 23,00 | 23,00 | 23,00 |
| Härte | | | |
| Anfang | 4,01 | 4,41 | 2,99 |
| Struktur Anfang | | | |
| Oberfläche | matt, sehr weiß | matt | glänzend matt |
| Abrieb | bröckelig | creamig weiß, fein | weiß leicht bröckelig |
| geschnittene Fläche | matt leicht bröckelig | leicht bröckelig | fein bröckelig |

**Tabelle 16: AP Stift silikonfrei**

| **Inhaltsstoffe** | **1** | **2** | **3** |
|---|---|---|---|
| Sebacinsäure-di-C16/18-ester | 14,70 | | 14,70 |
| Lanette® 18 | | 14,70 | |
| Cutina® HR | 3,70 | 3,70 | 3,70 |
| Cetiol® B | 29,35 | 29,35 | 17,50 |
| Synfluid® 2 cSt | 29,35 | 29,35 | |
| Rezal® 36GP | 22,90 | 22,90 | 22,90 |
| Arlamol® HD | | | 17,50 |
| Cetiol® OE | | | 9,00 |
| Cetiol® S | | | 14,70 |

| **Anhang** | | | |
|---|---|---|---|
| 1) | Arlamol® HD | 8) | Lanette^{®} 18 |
| | INCI: Heptamethylnonane | | INCI: Stearyl Alcohol |
| | Hersteller: Uniqema | | Hersteller: Cognis Deutschland GmbH & Co. KG |
| 2) | Cetiol® B | 9) | Rezal^{®} 36 GP |
| | INCI: Dibutyl Adipate Hersteller: Cognis Deutschland GmbH & | | INCI: Aluminium Zirconium Tetrachlorohydrex GLY |
| | Co. KG | | Hersteller: Reheis |
| 3) | Cetiol® OE | 10) | Synfluid® 2 cSt |
| | INCI: Dicaprylyl Ether | | INCI: Hydrogenated Didecene |
| | Hersteller: Cognis Deutschland GmbH & Co. KG | | Hersteller: Chevron |
| 4) | Cetiol® S | | |
| | INCI: Diethylhexylcyclohexane | | |
| | Hersteller: Cognis Deutschland GmbH & Co. KG | | |
| 5) | Cutina^{®} HR | | |
| | INCI: Hydrogenated Castor Oil | | |
| | Hersteller: Cognis Deutschland GmbH & Co. KG | | |
| 6) | Dow Corning^{®} 245 | | |
| | INCI: Cyclomethicone | | |
| | Hersteller: Dow Corning | | |
| 7) | Edenor^{®} OSSG | | |
| | INCI: 12-Hydroxystearic Acid | | |
| | Hersteller: Cognis Deutschland GmbH & Co. KG | | |

## Patentansprüche

1. Wasserfreie kosmetische Zusammensetzung mit einem Schmelzpunkt von wenigstens 30°C enthaltend
(a) wenigstens einen Dicarbonsäurediester einer C6-C18-Dicarbonsäure mit C12-C22-Fettalkoholen
(b) wenigstens eine Ölkomponente
(c) wenigstens ein anorganisches adstringierendes Salz, einen Deodorantwirkstoff oder ein Pigment.

2. Zusammensetzung gemäß Anspruch 1, **dadurch gekennzeichnet, dass** als Pigment wenigstens eine eisenoxidhaltige Verbindung enthalten ist.

3. Zusammensetzung gemäß Anspruch 1, **dadurch gekennzeichnet, dass** sie
(a) 5 - 40 Gew.-% wenigstens eines Diesters einer C6-C18-Dicarbonsäure mit C12-C22-Fettalkoholen
(b) 30 - 80 Gew.-% wenigstens einer Ölkomponente
(c) 10- 40 Gew.-% wenigstens eines anorganischen adstringierenden Salzes enthält.

4. Zusammensetzung gemäß wenigstens einem der Ansprüche 1 bis 3, **dadurch gekennzeichnet, dass** die Dicarbonsäurediester einen Schmelzpunkt von wenigstens 50 °C aufweisen, vorzugsweise von 50 - 55 °C.

5. Zusammensetzung gemäß wenigstens einem der Ansprüche 1 bis 4, **dadurch gekennzeichnet, dass** die Dicarbonsäure-Komponente ausgewählt ist aus den unverzweigten, gesättigten C6-C18-Dicarbonsäuren, vorzugsweise aus den unverzweigten, gesättigten C9-C18-Dicarbonsäuren, insbesondere den C12-C18-Dicarbonsäuren.

6. Zusammensetzung gemäß wenigstens einem der Ansprüche 1 bis 5, **dadurch gekennzeichnet, dass** die Alkohol-Komponente ausgewählt ist aus den unverzweigten, gesättigten C16-C22-Fettalkohlen.

7. Zusammensetzung nach wenigstens einem der Ansprüche 1 und 3 bis 6, **dadurch gekennzeichnet, dass** das anorganisches schweißhemmendes Salz ausgewählt ist aus den adstringierenden Aluminium-Verbindungen, Aluminium-Zirkonium-Verbindungen oder Zinksalzen.

8. Zusammensetzung nach wenigstens einem der Ansprüche 1 bis 7, **dadurch gekennzeichnet, dass** wenigstens eine Ölkomponente ausgewählt aus den Dialkyletherm, den Dialkylcarbonaten, den Kohlenwasserstoffe oder einem beliebigen Gemisch dieser Substanzen enthalten ist.

9. Verwendung von Dicarbonsäurediester einer C6-C18-Dicarbonsäure mit C12-C22- Fettalkoholen in Antitranspirant-Zusammensetzungen.

10. Verwendung von Dicarbonsäurediester einer C6-C18-Dicarbonsäure mit C12-C22- Fettalkoholen gemäß Anspruch 9 zur Herstellung wasserfreier Antitranspirant-Zusammensetzungen.

11. Verwendung von Dicarbonsäurediester einer C6-C18-Dicarbonsäure mit C12-C22- Fettalkoholen zur Verbesserung der Konsistenz von Antitranspirant- und Deostiften.

12. Verwendung von Dicarbonsäurediester einer C6-C18-Dicarbonsäure mit C12-C22- Fettalkoholen in Make-up- und Lippenstiften.

## Claims

1. Water-free cosmetic composition with a melting point of at least 30°C containing
(a) at least one dicarboxylic acid diester of a C6-C18 dicarboxylic acid with C12-C22 fatty alcohols,
(b) at least one oil component,
(c) at least one inorganic astringent salt, a deodorant component or a pigment.

2. Composition as claimed in claim 1, **characterized in that** at least one compound containing iron oxide is present as the pigment.

3. Composition as claimed in claim 1, **characterized in that** it contains
(a) 5 to 40% by weight of at least one diester of a C6-C18 dicarboxylic acid with C12-C22 fatty alcohols,
(b) 30 to 80% by weight of at least one oil component,
(c) 10 to 40% by weight of at least one inorganic astringent salt.

4. Composition as claimed in at least one of claims 1 to 3, **characterized in that** the dicarboxylic acid diesters have a melting point of at least 50°C and preferably of 50 to 55°C.

5. Composition as claimed in at least one of claims 1 to 4, **characterized in that** the dicarboxylic acid component is selected from unbranched, saturated C6-C18 dicarboxylic acids, preferably from unbranched, saturated C9-C18 dicarboxylic acids and, more particularly, from C12-C18 dicarboxylic acids.

6. Composition as claimed in at least one of claims 1 to 5, **characterized in that** the alcohol component is selected from unbranched, saturated C16-C22 fatty alcohols.

7. Composition as claimed in at least one of claims 1 and 3 to 6, **characterized in that** the inorganic perspiration-inhibiting salt is selected from astringent aluminium compounds, aluminium-zirconium compounds or zinc salts.

8. Composition as claimed in at least one of claims 1 to 7, **characterized in that** at least one oil component is selected from dialkyl ethers, dialkyl carbonates, hydrocarbons or a mixture of these substances.

9. Use of dicarboxylic acid diesters of a C6-C18 dicarboxylic acid with C12-C22 fatty alcohols in antiperspirant compositions.

10. Use of dicarboxylic acid diesters of a C6-C18 dicarboxylic acid with C12-C22 fatty alcohols as claimed in claim 9 for the production of water-free antiperspirant compositions.

11. Use of dicarboxylic acid diesters of a C6-C18 dicarboxylic acid with C12-C22 fatty alcohols for improving the consistency of antiperspirant and deodorant sticks.

12. Use of dicarboxylic acid diesters of a C6-C18 dicarboxylic acid with C12-C22 fatty alcohols in make-up sticks and lipsticks.

## Revendications

1. Composition cosmétique anhydre ayant un point de fusion d'au moins 30 °C, contenant
(a) au moins un diester d'acide dicarboxylique d'un acide dicarboxylique en C₆-C₁₈ avec des alcools gras en C₁₂-C₂₂
(b) au moins un composant huileux
(c) au moins un sel inorganique astringent, un principe actif déodorant ou un pigment.

2. Composition selon la revendication 1, **caractérisée en ce que** comme pigment est contenu au moins un composé contenant de l'oxyde de fer.

3. Composition selon la revendication 1, **caractérisée en ce qu'**elle contient
(a) 5 - 40 % en poids d'au moins un diester d'un acide dicarboxylique en C₆-C₁₈ avec des alcools gras en C₁₂-C₂₂
(b) 30 - 80 % en poids d'au moins un composant huileux
(c) 10 - 40 % en poids d'au moins un sel inorganique astringent.

4. Composition selon au moins l'une quelconque des revendications 1 à 3, **caractérisée en ce que** les diesters d'acides dicarboxyliques présentent un point de fusion d'au moins 50 °C, de préférence de 50 - 55 °C.

5. Composition selon au moins l'une quelconque des revendications 1 à 4, **caractérisée en ce que** le composant acide dicarboxylique est choisi parmi les acides dicarboxyliques en C₆-C₁₈ saturés non ramifiés, de préférence parmi les acides dicarboxyliques en C₉-C₁₈ saturés non ramifiés, en particulier les acides dicarboxyliques en C₁₂-C₁₈.

6. Composition selon au moins l'une quelconque des revendications 1 à 5, **caractérisée en ce que** le composant alcool est choisi parmi les alcools gras en C₁₆-C₂₂ saturés, non ramifiés.

7. Composition selon au moins l'une quelconque des revendications 1 et 3 à 6, **caractérisée en ce que** le sel inorganique antisudoral est choisi parmi les composés d'aluminium, composés d'aluminium-zirconium ou sels de zinc, astringents.

8. Composition selon au moins l'une quelconque des revendications 1 à 7, **caractérisée en ce que** ledit au moins un composant huileux est choisi parmi les éthers dialkyliques, les carbonates de dialkyle, les hydrocarbures ou un mélange quelconque de ces substances.

9. Utilisation de diesters d'acides dicarboxyliques d'un acide dicarboxylique en C₆-C₁₈ avec des alcools gras en C₁₂-C₂₂, dans des compositions antisudorales.

10. Utilisation de diesters d'acides dicarboxyliques d'un acide dicarboxylique en C₆-C₁₈ avec des alcools gras en C₁₂-C₂₂ selon la revendication 9, pour la préparation de compositions antisudorales anhydres.

11. Utilisation de diesters d'acides dicarboxyliques d'un acide dicarboxylique en C₆-C₁₈ avec des alcools gras en C₁₂-C₂₂, pour l'amélioration de la consistance de bâtons déodorants et antisudoraux.

12. Utilisation de diesters d'acides dicarboxyliques d'un acide dicarboxylique en C₆-C₁₈ avec des alcools gras en C₁₂-C₂₂ dans des bâtons pour les lèvres et de maquillage.
